# EUROPÄISCHE PATENTANMELDUNG

(11) **EP 2 815 737 A1**
(43) Veröffentlichungstag der Anmeldung: **24.12.2014**
(21) Anmeldenummer: 14181865.8
(22) Anmeldetag: 19.06.2008
(51) Int. Cl.: A61K 8/02, A61K 8/04, A61K 8/36, A61K 8/37, A61Q 17/04, A61Q 19/00, A61Q 19/10, A61K 9/12, A61K 9/127

(54) **DMS (Derma Membrane Structure) in Schaum-Cremes**

(30) Priorität: 19.06.2007 EP 07110571
(62) Teilanmeldung aus: 08761225.5
(71) Anmelder: Neubourg Skin Care GmbH & Co. KG, 48268 Greven (DE)
(72) Erfinder: Dr. Neubourg, Thomas, 59368 Werne (DE)
(74) Vertreter: Maiwald Patentanwalts GmbH

(57) **Zusammenfassung**

Schaumformulierung umfassend eine Emulsion, umfassend eine Ölphase und eine Wasserphase, wobei die Ölphase mindestens eine membranbildende Substanz umfasst, die in der Schaumformulierung eine lamellar angeordnete Membran ausbildet.

## Beschreibung

### Technisches Gebiet der Erfindung

Die vorliegende Erfindung betrifft kosmetische und dermatologische Schaumformulierungen, insbesondere Schaumcremes, auf der Basis von Emulsionen insbesondere vom Typ Öl-in-Wasser, wobei die Ölphase mindestens eine membranbildende Substanz umfasst, die in der Schaumformulierung eine lamellar angeordnete Membran ausbildet.

### Hintergrund der Erfindung

### 1. Emulsionen

Unter Emulsion versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (liphophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilien Molekülteil in ionische (anionische, kationische und amphotere) und nicht-ionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nicht-ionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Der Begriff "Emulgator" bzw. "herkömmlicher Emulgator" ist im Stand der Technik bekannt. Herkömmliche Emulgatoren und ihre Verwendung werden beispielsweise in den Publikationen: Pflegekosmetik, 4. Auflage, Wissenschaftliche Verlagsgesellschaft mbH Stuttgart, Seiten 151 bis 159, und Fiedler Lexikon der Hilfsstoffe, 5. Auflage Editio Cantor Verlag Aulendorf, Seiten 97 bis 121, beschrieben.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer herkömmlichen Emulsion ist die richtige Auswahl der Emulgatoren. Dabei sind die Charakteristika aller im System enthaltenen Stoffe zu berücksichtigen. Betrachtet man z.B. Hautpflegeemulsionen, so können polare Ölkomponenten wie z. B. UV-Filter zu Instabilitäten führen. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar, welche in den unterschiedlichsten Anwendungsgebieten eingesetzt werden. So stehen für die Pflege der Haut, insbesondere für die Rückfettung trockener Haut, eine Vielzahl von Produkten - wie Lotionen und Cremes - zur Verfügung. Ziel der Hautpflege ist es, den durch tägliches Waschen verursachten Fett- und Wasserverlust der Haut auszugleichen. Außerdem sollen Hautpflegeprodukte vor Umwelteinflüssen - insbesondere vor Sonne und Wind - schützen und die Hautalterung verzögern.

Kosmetische Emulsionen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Auch zur Reinigung der Haut und Hautanhangsgebilde finden Emulsionen in Form von Reinigungsemulsionen Verwendung. Sie werden meist der Gesichtsreinigung und insbesondere zur Entfernung von dekorativer Kosmetik eingesetzt. Derartige Reinigungsemulsionen haben - im Gegensatz zu anderen Reinigungszubereitungen wie beispielsweise Seife - den Vorteil, besonders hautverträglich zu sein, da sie in ihrer lipophilen Phase pflegende Öle und/oder unpolare Wirkstoffe - wie z.B. Vitamin E - enthalten können.

### 2. Emulgatorfreie Emulsionen

Seit Jahrzehnten bilden herkömmliche Emulgatoren die Grundlage für die Entwicklung von Hautpflegepräparaten. Emulgatoren wurden als Hilfsstoffe zur Herstellung und vor allem zur Stabilisierung von Emulsionen eingesetzt. In jüngerer Zeit gibt es Hinweise, dass die Verwendung von Emulgatoren in Hautpflegepräparaten z.B. bei empfindlicher Haut zu Problemen führen kann, da die Emulgatoren typischerweise die Integrität der natürlichen Hautbarriere stören und es so bei der Hautreinigung zu einem Verlust von natürlichen Barrierestoffen der Haut kommen kann. Der Verlust der natürlichen Barrierestoffe kann erhöhte Rauigkeit, trockene Haut, Rissigkeit und Abnutzungsekzeme verursachen.

Weiterhin führt die Verwendung von Emulgatoren normalerweise dazu, dass die lamellaren Strukturen der Lipidbarriere in vesikuläre Strukturen, wie z.B. Mizellen oder Mischmizellen umgewandelt werden. Diese Vesikel "zerstören" zumindest einen Teil der Barriereschicht der Haut und erhöhen dadurch lokal die Durchlässigkeit der Barriereschicht-Membran. Durch diese Öffnung der Barriereschicht der Haut wird zumindest vorübergehend der transepidermale Wasserverlust (TEWL) erhöht und gleichzeitig kommt es zu einer Abnahme des Feuchtigkeitsbindevermögens der Haut. Bei kontinuierlicher Anwendung von Pflegepräparaten mit herkömmlichen Emulgatoren kann es sogar dazu führen, dass die Haut nicht in der Lage ist, ihre Schutzfunktion aufrecht zu erhalten.

Eine besondere Form der Emulsion stellen emulgatorfreie Emulsionen dar. Diese sind frei von Emulgatoren im herkömmlichen Sinne, d.h. von amphiphilen Substanzen mit niedrigen Molmassen (d.h. Molmasse < 5000), die in geeigneten Konzentrationen Mizellen und/oder andere flüssigkristalline Aggregate ausbilden können. Die IUPAC definiert den Begriff "Emulgator" wie folgt: Emulgatoren (d.h. herkömmliche Emulgatoren) sind grenzflächenaktive Substanzen. Sie halten sich bevorzugt in der Grenzfläche zwischen Öl- und Wasserphase auf und senken dadurch die Grenzflächenspannung. Emulgatoren erleichtern bereits bei niedriger Konzentration die Emulsionsbildung. Darüber hinaus vermögen diese Substanzen die Stabilität von Emulsionen zu verbessern, indem sie die Geschwindigkeit der Aggregation und/oder der Koaleszenz verringern. Nach einer im interdisziplinären Konsens zwischen Pharmazeuten, Dermatologen und anderen Fachleuten verabschiedeten Begriffsdefinition der Gesellschaft für Dermopharmazie (http://www.dermotopics.de/german/ ausgabe_1_03_d/emulgatorfrei_1_2003_d.htm) kann eine Formulierung als "emulgatorfrei" bezeichnet werden, wenn sie statt mit Emulgatoren im engeren Sinne (herkömmliche Emulgatoren) mit grenzflächenaktiven Makromolekülen (Molmasse von über 5000) stabilisiert ist.

Zur Stabilisierung pharmazeutischer und kosmetischer Emulsionen werden überwiegend sogenannte echte Emulgatoren, d.h. herkömmliche Emulgatoren im Sinne der vorliegenden Beschreibung, eingesetzt, die ihrer Struktur und ihrem physikalisch-chemischen Verhalten nach zur Stoffklasse der Tenside zählen. Sie zeichnen sich durch einen amphiphilen Aufbau und die Fähigkeit zur Mizellenassoziation aus. Verbindungen oder Stoffgemische, die statt zur Mizellenbildung zur Ausbildung einer lamellar angeordneten Membran im Sinne der vorliegenden Erfindung führen, gelten jedoch nicht als herkömmliche Emulgatoren. Beispiele für solche Verbindungen sind etwa Phospholipide, wie etwa Lecithine, Sphingolipide, Ceramide, Cholesterin, Fettalkohole, Fettsäuren sowie deren Mono- und/oder Diester, sowie Sterole, etc., wenn sie unter bestimmten Bedingungen, wie nachstehend beschrieben, dispergiert werden. Derartige Stoffgemische können ferner Triglyceride (nicht hydrophil und lipophil), Squalen (nicht hydrophil und lipophil), oder Squalan (nicht hydrophil und lipophil) enthalten. Bevorzugte Beispiele für membranbildende Substanzen der vorliegenden Erfindung sind Phospholipide, Sphingolipide, Ceramide, Cholesterin, Fettalkohole, Fettsäuren sowie deren Mono- und/oder Diester, und Sterole. Diese Verbindungen, z.B Phospholipide, sind im Gegensatz zu typischen Emulgatoren, insbesondere Tensiden mit einem vergleichbaren HLB-Wert von etwa 10, in Wasser unlöslich. Sie bilden normalerweise alleine keine Mizellen oder hexagonale flüssigkristalline Phasen. Sie bilden oberhalb der Phasenübergangstemperatur in Wasser spontan ausschließlich große multilamellare Vesikel (large multilamellar vesicles; LUVs). Unterhalb der Phasenüberganstemperatur lassen sie sich unter hohem Energieeintrag in Wasser dispergieren und bilden lamellare Strukturen. Die genannte Phasenübergangstemperatur gibt hierbei die Temperatur an, bei der eine gelförmige Phase in eine flüssigkristalline Phase übergeht. Unterhalb der Phasenübergangstemperatur liegt eine Gelphase vor, oberhalb davon liegt eine flüssigkristalline Phase vor. Die Phasenübergangstemperaturen variieren je nach Zusammensetzung (gesättigt/ungesättigt; kurz/lang) und liegen beispielsweise bei Phospholipiden typischerweise zwischen 10°C und 70°C. Für ein gegebenes System kann die Phasenübergangstemperatur leicht mittels DSC gemessen werden.

Membranbildende Substanzen enthalten typischerweise ebenso lipophile und hydrophile Molekülteile. Die Fähigkeit einer membranbildenden Substanz im Gegensatz zu Mizellen lamellare Strukturen zu bilden, hängt aber insbesonderen von der optimalen Fläche aₒ (Grenzfläche Kohlenstoff/Wasser), dem Volumen V und der kritischen Kettenlänge l_{c} ab (Israelachvili, Jacob N.: "Intermolecular and Surface Forces: With Applications to Colloidal and Biological Systems". 2nd Edition Academic Press, London, UK, 1992).

Weiterhin ist unter Umständen notwendig, spezielle Herstellungsbedingungen zu wählen, damit ein System lamellare Strukturen ausbildet. Diese werden im folgenden für die erfindungsgemäßen Systeme noch näher beschrieben. Zwar sind Systeme in der Technik bekannt, bei denen sich mizellare Strukturen unter geeigneten Bedingungen in lamellare Strukturen überführen lassen, jedoch gibt es ebenfalls Systeme, bei denen keine Phasentransformation in eine andere Phase wie z.B. eine mizellare, hexagonale Phase, etc. möglich ist. Wiederum andere Systeme erlauben unter geeigneten Bedingungen die Ausbildung einer lamellaren Phase; jedoch führt eine Konzentrationsveränderung nicht dazu, dass andere Mesophasen ausgebildet werden.

Lamellare Strukturen bilden sich somit unter wohl definierten Bedingungen und sind nicht beliebig durch Änderung der Konzentration in andere Mesophasen wie z.B. mizellare Strukturen überführbar. Bei wasserlöslichen Tensiden (Emulgatoren) bilden sich Mizellen, hexagonale und lamellare flüssigkristalline Phasen in Abhängigkeit von der Tensidkonzentration. Dabei ist es auch möglich, dass in Abhängigkeit von der Konzentration Mischungen der verschiedenen Zustände (hexagonal und lamellar) im Gleichgewicht nebeneinander vorliegen. Im Gegensatz dazu sind membranbildende Substanzen der vorliegenden Erfindung typischerweise in Wasser unlöslich. Bei diesen wasserunlöslichen Lipiden, wie z.B. Phospholipiden, liegen in der Regel liposomale Strukturen nicht neben lamellaren Strukturen vor, sondern entweder die eine Struktur oder die andere.

Ein Beispiel für emulgatorfreie Emulsionen sind Pickering-Emulsionen. Pickering Emulsionen sind feststoffstabilisierte Emulsionen, in denen die feinstverteilten Feststoffteilchen die Emulsion stabilisieren, so dass weitestgehend auf herkömmliche Emulgatoren verzichtet werden kann. Dabei kommt es zu einer Anreicherung des festen Stoffes an der Phasengrenze Öl/Wasser in Form einer Schicht, wodurch ein Zusammenfließen der dispersen Phase verhindert wird. Solche geeigneten Feststoffemulgatoren sind partikuläre anorganische oder organische Feststoffe, die sowohl von lipophilen als auch von hydrophilen Flüssigkeiten benetzbar sind. Bevorzugt werden in den Pickering Emulsionen z.B. Titandioxid, Zinkoxid, Siliziumdioxid, Fe₂O₃, Veegum, Bentonit oder Ethylcellulose als Feststoffe eingesetzt.

Jedoch können auch diese Feststoffemulgatoren bei empfindlicher Haut zu Reizungen führen oder sogar Allergien auslösen.

Es werden heute bereits Cremegrundlagen eingesetzt, die mit einer Vielzahl von natürlichen bzw. hautähnlichen Inhaltsstoffen arbeiten, die eine bessere Hautverträglichkeit insbesondere bei empfindlicher Haut versprechen. Dabei hat sich gezeigt, dass durch die Verwendung von hautähnlichen Inhaltsstoffen eine verbesserte Hautpflege erzielen lässt. So werden in diesen Cremegrundlagen z.B. einige Bestandteile der natürlichen Hautlipide wie z.B. Triglyceride durch (pflanzliche) Caprylsäure/Caprinsäuretriglyceride , Squalen durch (pflanzliches) Squalan, Ceramide durch Ceramide 3 (aus Hefe), Cholesterol durch (pflanzliche) Phytosterole und Phospholipide durch (pflanzliche) Phospholipide ersetzt.

Bevorzugt wird bei diesem Konzept auf viele übliche Hilfsstoffe wie Duftstoffe, Farbstoffe, komedogene Lipide (z.B. Mineralöle), Konservierungsmittel und physiologisch bedenkliche Emulgatoren verzichtet, da diese potenziell sensibilisierend sind und zu Irritationen der Haut führen können.

Diese Formulierungen werden bevorzugt ohne herkömmliche Emulgatoren zubereitet, um die oben genannten Nachteile von herkömmlichen Emulgatoren zu vermeiden.

Ohne sich auf eine bestimmte Theorie zu stützen, wird angenommen, dass die besondere Wirkung dieser spezifisch zusammengesetzten Membranlipide mit der lamellaren Struktur zusammenhängt. Der Verzicht auf herkömmliche Emulgatoren verhindert, dass Mizellen oder Vesikel gebildet werden, so dass die lamellare Struktur der ausgebildeten Membran in der Emulsion erhalten bleibt. Diese lamellare Struktur ist dem (physikalischen) Aufbau und der (chemischen) Zusammensetzung der natürlichen epidermalen Hautlipide nachempfunden, die sich bevorzugt als Kittsubstanz zwischen den Zellen (Corneozyten) des Stratum corneums finden.

Systeme, die auf spezifisch zusammengesetzte Membranlipide zurückgreifen, die eine lamellare Struktur der Membran aufweisen, sind unter dem Begriff "DMS®" (Derma Membrane Structure) in der Technik bekannt.

### 3. Schaumformulierungen

Eine besondere Anwendungsform kosmetischer und oder dermatologischer Emulsionen ist die Applikation als Schäume. Schaumformulierungen haben den Vorteil, sich leicht auf der Haut verteilen zu lassen. Die schaumige Konsistenz wird als angenehm empfunden und die Produkte hinterlassen in der Regel ein gutes Hautgefühl. Insbesondere wirkt sich aber auch die physikalische Struktur des Schaumes positiv auf die Hautschutzfunktion aus. Schäume sind komplizierte physikalische Gebilde, die einer besonderen Abstimmung der den Schaum bildenden Komponenten bedürfen. Schäume werden generell durch Versprühen einer Emulsionsformulierung oder einer wässrigen Tensid-(Stabilisator-)lösung erhalten.

Beispielsweise wird die mit Treibgas versetzte Emulsion aus einem unter Druck stehenden Behältnis abgegeben (solche Systeme werden in der Literatur und Patentliteratur auch als Aerosolschäume bezeichnet). Dabei expandiert das unter Druck stehende Gemisch aus Emulsion und Treibgas und bildet die Schaumbläschen. Insbesondere kommt es zu einer Expansion der dispersen Ölphase, in welcher das öllösliche Gas gelöst ist. Schäume können aber auch mit Hilfe anderer Systeme erzeugt werden, wie beispielsweise Pumpsprays.

Abgestimmte Schaumformulierungen weisen eine stabile zwei- oder mehrphasige polydisperse Struktur bei der Applikation auf, die auf der Haut eine Netzstruktur, vergleichbar mit einer Membran bildet. Solche Netzstrukturen haben den Vorteil, dass diese eine Schutzfunktion, beispielsweise vor Kontakt mit Wasser ausbilden, jedoch einen ungehinderten Gasaustausch mit der Umgebung zulassen. Bei solchen Schäumen kommt es praktisch zu keiner Behinderung der *Perspiratio insensibiles* und keinem entsprechenden Wärmestau. Damit werden die positiven Eigenschaften einer Schutz- und Pflegefunktion mit einer unveränderten Hautatmung verbunden.

Bisher bekannte Schaumformulierungen enthalten herkömmliche Tenside/Emulgatoren, die für die Stabilisierung der Emulsion und für die darauf folgende Schaumstabilität sorgen.

Herkömmliche Emulgatoren bzw. Tenside werden jedoch, wie bereits zuvor diskutiert, immer wieder als Ursache für Unverträglichkeiten bei Hautpflegeprodukten genannt, wie z.B. eine Störung der Hautbarriere oder Mallorcaakne.

Daher besteht ein Bedarf an individuellen Hautpflegezusammensetzungen, die den Hautbedürfnissen besser gerecht werden, als herkömmliche Emulsionssysteme auf Emulgatorbasis und somit einen besseren Hautschutz und eine verbesserte Hautpflege bereitstellen.

Ein Einsatz von Cremegrundlagen, die eine lamellare Struktur in der Zusammensetzung angelehnt an die membranbildenden epidermalen Lipide aufweisen, ist in Schaumformulierungen bislang nicht beschrieben worden.

Aufgabe der vorliegenden Erfindung ist es, verbesserte Schaumformulierungen, insbesondere bessere Schaumcremes, bereitzustellen, die die oben genannten Nachteile der Formulierungen des Standes der Technik umgeht.

### Zusammenfassung der Erfindung

Die Anmelderin hat überraschenderweise erkannt, dass sich Emulsionen, die eine Ölphase und eine Wasserphase umfassen, wobei die Ölphase mindestens eine membranbildende Substanz umfasst, die in der Schaumformulierung eine lamellar angeordnete Membran ausbildet, als Basis für Schaumformulierungen eignen. In einer bevorzugten Ausführungsform sind die Schaumformulierungen im Wesentlichen emulgatorfrei, d.h. sie enthalten im Wesentlichen keine herkömmlichen Emulgatoren, wobei die Substanz oder das Substanzgemisch, das zur Ausbildung einer lamellar angeordneten Membran führt, nicht als herkömmlicher Emulgator gilt. So ist es beispielsweise im Fachgebiet Verkehrsauffassung, z.B. das Handelsprodukt Physiogel® Creme, die DMS®-Konzentrat enthält, als "Emulgatorfrei" zu bezeichnen.

Die membranbildenden Substanzen und Substanzgemische gemäß der vorliegenden Erfindung sind typischerweise in Wasser unlöslich, während herkömmliche Emulgatoren, insbesondere Tenside mit einem vergleichbaren HLB-Wert von etwa 10, in der Regel wasserlöslich sind. Ferner sind die wasserunlöslichen membranbildenden Substanzen gemäß der vorliegenden Erfindung nicht in der Lage spontan Öle zu emulgieren, während herkömmliche Emulgatoren, insbesondere jene mit hohem HLB-Wert, spontan Öle emulgieren können. Herkömmliche Emulgatoren mit niedrigem HLB-Wert sind im Gegensatz zu membranbildenden Substanzen gemäß der vorliegenden Erfindung, z.B. Phospholipiden, nicht in der Lage alleine lamellare Strukturen oder Liposomen zu bilden. Eine Besonderheit von membranbildenden Substanzen der vorliegenden Erfindung im Gegensatz zu herkömmlichen Emulgatoren ist, dass beispielsweise Phospholipide einen HLB von 10 aufweisen und dennoch wasserunlöslich sind.

Vorzugsweise weisen die membranbildenden Substanzen der Erfindung einen HLB-Wert von größer als 8 auf, mehr bevorzugt von 9 bis 11, und am meisten bevorzugt von 9,5 bis 10,5.

Gemäß der vorliegenden Erfindung werden die positiven Eigenschaften der Schaumformulierungen mit denen der Emulsionen, in denen die Ölphase mindestens eine membranbildende Substanz umfasst, die in der Schaumformulierung eine lamellar angeordnete Membran ausbildet, verbunden. Dabei lassen sich insbesondere Schaumformulierungen herstellen, die die positiven Eigenschaften des Schaums, nämlich die physikalische Struktur und die angenehme Anwendbarkeit mit einer guten Hautverträglichkeit kombinieren. Diese Eigenschaft erlaubt den Einsatz der Schaumformulierungen für kosmetische und dermatologische Formulierungen, die bei empfindlichen Hauttypen eingesetzt werden. Es wird dabei Verträglichkeit und Anwendungskomfort vorteilhaft miteinander verbunden. Die für die Hautverträglichkeit wichtige lamellare Struktur der mindestens einen membranbildenden Substanz ist in Schaumformulierungen des Standes der Technik nicht berücksichtigt worden.

Dabei ist es nicht selbstverständlich, dass diese Emulsionen beim Verschäumen zu stabilen Schaumprodukten führt. Schäume werden, wie bereits erwähnt, beispielsweise durch Einarbeiten von Treibgasen in O/W-Emulsionssystemen erhalten. Verdampft bei dem Verschäumen das z.B. in der dispersen Ölphase gelöste Treibgas, bildet sich ein Schaum (Gas in Flüssigdispersion). Beim Verdampfen bzw. Expandieren des in der dispersen Ölphase gelösten Treibgases kommt es zu einer Dilatation der dispersen Ölphase. Es ist nun überraschend gefunden worden, dass es beim Verschäumen der erfindungsgemäßen Schaumformulierungen nicht zum Brechen der Zubereitung kommt und ein geeigneter Schaum ausgebildet wird. Der gebildete Schaum ist stabil genug, um z.B. auf die Haut aufgetragen zu werden.

Die Erfindung betrifft Schaumformulierungen, umfassend eine Ölphase und eine Wasserphase, wobei die Ölphase mindestens eine membranbildende Substanz umfasst, die in der Schaumformulierung eine lamellar angeordnete Membran ausbildet.

Bevorzugt betrifft die Erfindung Schaumformulierungen, die auf Basis natürlicher bzw. hautähnlicher Inhaltsstoffe arbeiten, die eine bessere Hautverträglichkeit ermöglichen.

Ferner betrifft die Erfindung die Verwendung von Schaumformulierungen auf Basis von Emulsionen als Träger für Wirkstoffe, als Hautpflegemittel, als Hautreinigungsmittel oder als Sonnenschutzmittel. Die Schaumformulierung kann daher als Kosmetikum, Medizinprodukt oder Arzneimittel eingesetzt werden.

Außerdem umfasst die Erfindung ein Verfahren zur Herstellung von Schaumformulierungen basierend auf Emulsionen, in denen die Ölphase eine membranbildende Substanz umfasst, die in der Schaumformulierung eine lamellar angeordnete Membran ausbildet. Das Verfahren umfasst die Schritte:
a) Herstellen einer Emulsion bevorzugt vom Typ Öl in Wasser
b) Abfüllen der Emulsion und Treibgas in einen Druckbehälter, oder
c) Abfüllen der Emulsion in einen anderen als einen Druckbehälter, der bei Abgabe der Emulsion einen Schaum erzeugt.

### Beschreibung der Abbildung

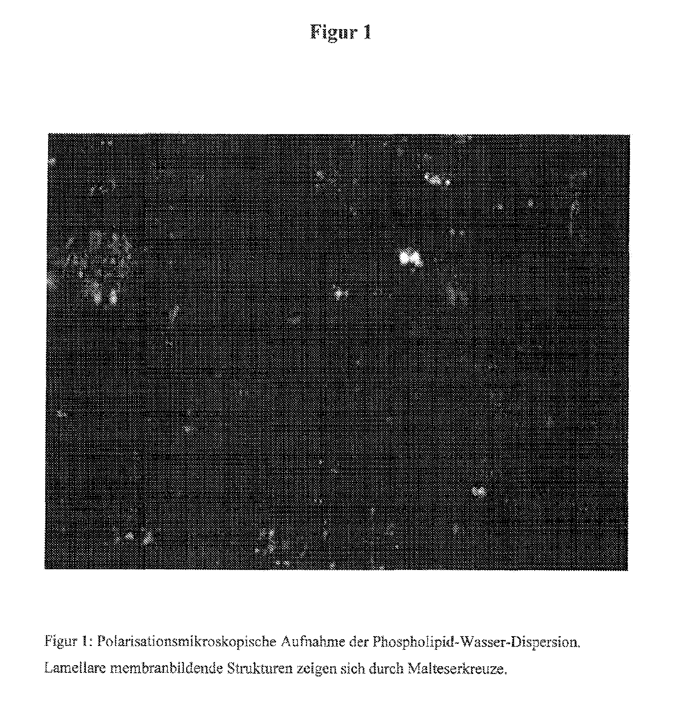

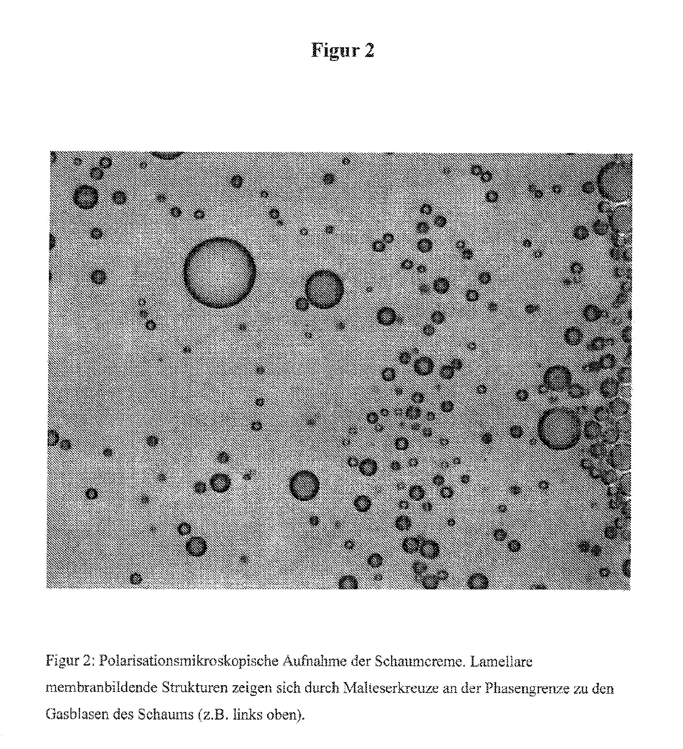

Figuren 1-3 zeigen polarisationsmikroskopische Aufnahmen der Schaumformulierung aus Beispiel 3.

Figur 1 zeigt lamellare membranbildende Strukturen, die durch sogenannte Malteserkreuze erkennbar sind (insbesondere im linken, oberen Bildbereich).

In Figur 2 ist zudem die Gasphase der Schaumformulierung in Form von Gasbläschen zu erkennen. Malteserkreuze zeigen sich insbesondere an der Phasengrenze zur Gasphase.

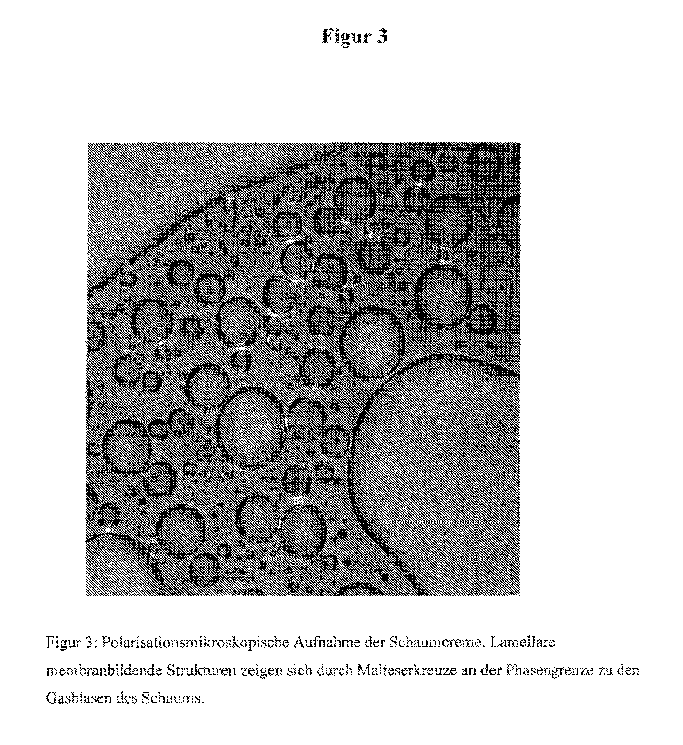

Ebenso sind in Figur 3 Malteserkreuze an der Phasengrenze zu den Gasblasen des Schaums erkennbar.

### Detaillierte Beschreibung der Erfindung

Gemäß der vorliegenden Erfindung sind Schaumformulierungen Formulierungen, insbesondere Emulsionen, die zur Erzeugung von Schaum sinnfällig hergerichtet sind. Die Formulierungen können insbesondere entweder mit Treibgas in einen Druckbehälter abgefüllt sein oder ohne Treibgas in einen anderen Behälter als einen Druckbehälter abgefüllt sein, der es ermöglicht, bei Abgabe der Formulierung/Emulsion einen Schaum zu erzeugen. Pumpspraybehälter können beispielsweise ebenfalls verwendet werden.

In einer bevorzugten Ausführungsform ist die Schaumformulierung eine Schaumcreme.

Gemäß der vorliegenden Erfindung sind im Wesentlichen emulgatorfreie Emulsionen solche Emulsionen, die nicht mehr als 1,5 Gew.-%, bevorzugt nicht mehr als 1,0 %, mehr bevorzugt nicht mehr als 0,5 % herkömmlicher Emulgatoren enthalten. Gemäß der Erfindung sind emulgatorfreie Emulsionen solche, die keine herkömmlichen Emulgatoren enthalten.

Gemäß der vorliegenden Erfindung ist eine membranbildende Substanz, die eine lamellar angeordnete Membran ausbildet, eine Substanz, die vorzugsweise zugleich einen hydrophilen sowie einen hydrophoben Molekülrest aufweist. Bevorzugt sind Substanzen wie z.B. Phospholipide, wie etwa Lecithine, Sphingolipide, Ceramide, Cholesterin, Fettalkohole, Fettsäuren sowie deren Mono- und/oder Diester, sowie Sterole, etc. Triglyceride (nicht hydrophil und lipophil), Squalen (nicht hydrophil und lipophil), Squalan (nicht hydrophil und lipophil), können ebenfalls in Stoffgemischen enthalten sein, die membranbildende Substanzen umfassen. Bevorzugte membranbildende Substanzen sind Phospholipide, Sphingolipide, Ceramide, Cholesterin, Fettalkohole, Fettsäuren sowie deren Mono- und/oder Diester, und Sterole. Solche Substanzen oder entsprechende Substanzgemische können auf geeignete Weise mit einer wässrigen Phase unter Ausbildung von lamellaren Membranen dispergiert werden. Dies kann beispielsweise durch Dispergieren unter hohem Energieeintrag (z.B. Hochdruckhomogenisation, Ultraschall) erreicht werden. Im Falle der Hochdruckhomgenisation werden dabei Drücke im Bereich von 50.000-250.000 Kilopascal (500-2500 bar), mehr bevorzugt von 100.000-150.000 Kilopascal (1000-1500) bar eingesetzt. In anderen Fällen ist ein hoher Energieeintrag zur Ausbildung lamellarer Membranstrukturen nicht zwingend notwendig (häufig etwa bei Verwendung von nicht-hydrierten Lecithinen mit niedriger Phasenübergangstemperatur und in Verbindung mit geeigneten Lipiden, wie z.B. Isopropylmristat). Die Verwendung von bestimmten Konzentraten, die eine lamellare Phase ausbilden, ist ebenfalls möglich.

Das Vorliegen von lamellaren Strukturen in der Dispersion kann der Fachmann leicht mit Hilfe im Stand der Technik bekannter Verfahren feststellen. Geeignete Messmethoden sind beispielsweise in Claus-Dieter Herzfeld et al. (Hrsg.), Grundlagen der Arzneiformlehre, Galenik 2, Springer Verlag, 1999, beschrieben. Besonders erwähnungswert ist hierbei die Methode der Polarisationsmikroskopie. Dabei werden zwei Polarisationsfolien in der sogenannten Kreuzstellung, bei der die Schwingungsebenen des erzeugten polarisierten Lichts senkrecht zueinander stehen über und unter dem zu untersuchenden Objekt angebracht. Die Schwingungsebene des eingestrahlten Lichts wird durch die Probe verändert, so dass ein Anteil des Lichts die zweite Polarisationsfolie passieren kann. Das Vorliegen von Lammellarphasen ist hier typischerweise durch sogenannte Malteserkreuze erkennbar.

Gemäß der vorliegenden Erfindung ist eine lamellar angeordnete Membran so angeordnet, dass sie einen Schichtaufbau besitzt, derart, dass die jeweils obere Schicht der Substanz zu einer unteren Schicht der Substanz zueinander ausgerichtet ist. Die Ausrichtung der einzelnen Substanzschichten erfolgt zueinander unabhängig von dem verwendeten Lösungsmittel in der Art, dass z.B. die hydrophilen Reste der Substanz nach außen weisen und die hydrophoben Reste zueinander nach innen ausgerichtet sind oder umgekehrt.

Orientieren sich zwei Schichten der Substanz in dem oben beschriebenen Sinne, so spricht man von einer Einfachmembran, während bei einer Übereinanderordnung von zwei weiteren Schichten diese lamellare Struktur als Doppelmembran bezeichnet wird. Gemäß dem vorliegenden Prinzip können noch weitere Schichten an die bereits vorliegenden (Doppel)-Membran assoziiert werden, was zu einem Mehrfachmembranaufbau führt. Gemäß der vorliegenden Erfindung kann die Membran als Einfachmembran, als Doppelmembran oder auch als Mehrfachmembran vorliegen.

Unter dem "wash out" Effekt wird ein Absenken der Hautfeuchtigkeit nach Beenden der Applikation der Pflegezusammensetzung unterhalb des Ausgangswertes verstanden.

Gemäß der vorliegenden Erfindung sind körperidentische Fette, im Körper vorkommende Fette pflanzlichen Ursprungs.

### Ölphase

Geeignete Komponenten, welche die Ölphase bilden können, können aus den polaren und unpolaren Lipiden oder deren Mischungen gewählt werden.

Die Ölphase der erfindungsgemäßen Formulierungen wird vorteilhaft gewählt aus der Gruppe der Phospholipide, wie etwa Lecithine, (Mono-, Di-, Tri-)-Glyceride (insbesondere Triglyceride, wie z.B. der Fettsäuretriglyceride), Sphingolipide, aus der Gruppe der Propylenglykol- oder Butylenglykol-Fettsäureester, aus der Gruppe der natürlichen Wachse, tierischen und pflanzlichen Ursprungs, aus der Gruppe der Esteröle, aus der Gruppe der Dialkylether und Dialkylcarbonate, aus der Gruppe der verzweigten und unverzweigten Kohlenwasserstoffe und Wachse sowie aus der Gruppe der zyklischen und linearen Silikonöle.

Die Schaumformulierungen gemäß der vorliegenden Erfindung ermöglichen aufgrund des lamellaren Membranaufbaus und der daraus resultierenden strukturelle Ähnlichkeit zum Aufbau der interzellulären lamellaren Lipidstruktur der epidermalen Lipide, insbesondere des *Stratum corneums,* eine verbesserte Pflegewirkung der Formulierung. Durch den analogen Aufbau der lamellaren Struktur der Haut wird eine leichtere Integration der Membran in die Haut erzielt. Die Integration führt ebenfalls zu einer Verbesserung, insbesondere der Stabilisierung und der Wiederherstellung, der Hautbarriere. Eine intakte Hautbarriere schützt die Haut vor zu hohem Feuchtigkeitsverlust. Eine Verbesserung der Hautbarriere kann ebenfalls eine verbesserte Hautglättung bewirken und den "wash-out" Effekt verringern, wodurch vorteilhaft ein besserer Langzeiteffekt im Vergleich zu herkömmlichen Schaumformulierungen erzielt wird.

Bevorzugte Schaumformulierungen der vorliegenden Erfindung verwenden "hautähnliche" Bestandteile, um die Ähnlichkeit der lamellar angeordneten Membran, die in der Schaumformulierung vorliegt, mit der Haut zu erzeugen. Besonders bevorzugte Ausführungsformen ersetzen dabei z.B. die in der Hornschicht vorliegenden natürlichen Glyceride (die Haut enthält überwiegend ein Gemisch aus Di- und Triglyceriden) durch z.B. (pflanzliche) Triglyceride, das Squalen durch z.B. das weniger oxidationsempfindliche Squalan, Ceramide durch Ceramide 3 (aus Hefe), Cholesterol durch (pflanzliche) Phytosterole und die Phospholipide durch (pflanzliche) Phospholipide ersetzt.

In einer bevorzugten Schaumformulierung der Erfindung umfasst die membranbildende Substanz ein Lipid, mehr bevorzugt ein Triglycerid und/oder Phospholipid. In einer besonders bevorzugten Schaumformulierung der Erfindung ist das Triglycerid Caprylsäure-/Caprinsäuretriglycerid und/oder das Phospholipid hydriertes Lecithin.

In einer weiteren bevorzugten Schaumformulierung der vorliegenden Erfindung kann die Formulierung ferner Lecithin, bevorzugt hydriertes Lecithin umfassen.

Die bevorzugten erfindungsgemäßen Schaumformulierungen können ferner weitere Bestandteile wie z.B. Stabilisatoren wie z.B. Alkohole oder Glykole enthalten sein. Bevorzugt sind Glykole, insbesondere Propylenglykol, Caprylylglykol oder Mischungen davon.

In bevorzugten Schaumformulierungen der Erfindung können weitere Bestandteile wie z.B. Butyrospermum Parkii (Sheabutter), Squalan, Gylceride, Ceramide, bevorzugt Ceramid 3 oder Mischungen der vorgenannten umfassen.

Eine bevorzugte Schaumformulierung der Erfindung umfasst eine im Wesentlichen emulgatorfreie Emulsion. Eine besonders bevorzugte Schaumformulierung der Erfindung ist emulgatorfrei. In einer bevorzugten Ausführungsform ist die Schaumformulierung frei von wasserlöslichen herkömmlichen Emulgatoren mit einem HLB-Wert von etwa 10. In einer bevorzugten Ausführungsform ist die Formulierung insbesondere frei von folgenden Verbindungen:
Carboxylate, wie z.B. Natriumstearat, Aluminiumstearat;
Sulfate, wie z.B.Na-Dodecylsulfat, Na-Cetylstearylsulfat, Na-Laurylethersulfat Sulfonat: Na-Dioctylsulfosuccinat;
quartäre Ammoniumverbindungen, wie z.B. Cetyltrimethylammoniumbromid, Benzalkoniumbromid;
Pyridinium-Verbindungen, wie z.B. Cetylpyrdiniumchlorid;
Betainen, wie z.B. Betainmonohydrat;
Macrogolfettsäureester, wie z.B. Macrogol-30-Stearat;
Glycerolfettsäureester, wie z.B. Glycerolmonostearat, Glycerolmonooleat, Glycerolmonoisostearat, Partialglyceride, mittelkettige Polyoxyethylensorbitan¬fettsäureester, wie z.B. Tween®, Polyoxyethylen-(20)-sorbitanmonostearat;
Sorbitanfettsäureester, wie z.B. Sorbitanlaurat, Sorbitanmonooleat, Sorbitanmonopalmitat, Sorbitanmonostearat, Sorbitantristearat, Sorbitansesquioleat; Saccharosefettsäureester, wie z.B. Saccharosemonostearat; Saccharosedistearat, Sacharosecocoat;
Macrogolfettalkoholether, wie z.B. Cetomacrogol 1000, Macrogolcetostearylether, Macrogololeylether, Lauromacrogol 400;
Sterinalkohole, wie z.B. Cholesterol, Wollwachs, acetylierter Wollwachs, hydrierter Wollwachs, Wollwachsalkohole;
Macrogolglycerolfettsäureester, wie z.B. Macrogol-1000-glycerol-Monooleat, Macrogol-1000-glycerol-monostearat, Macrogol-1500-glycerol-triricinoleat, Macrogol-300-glyceroltris(hydroxystearat), Macrogol-5-Glycerol-stearat, Macrogolglycerolhydroxystearat;
Polyglycerolfettsäurester, wie z.B. Triglyceroldiisostearat.

In der vorliegenden Erfindung wird die Ölphase, die eine zur Bildung von lamellar angeordneten Membranen geeignete Substanz oder ein solches Substanzgemisch umfasst, mit einer Wasserphase unter Bedingungen dispergiert, die zur Bildung einer lamellaren Phase führen. Falls notwendig geschieht dies beispielsweise durch Dispergieren unter hohem Energieeintrag, wie z.B. unter Ultraschall oder mittels Hochdruckhomogenisation, wobei Drücke von etwa 50.000 bis etwa 250.000 Kilopascal (etwa 500 bis etwa 2500 bar), vorzugsweise etwa 100.000 bis etwa 150.000 Kilopascal (etwa 1000 bis etwa 1500 bar) verwendet werden. In anderen Fällen, insbesondere bei Verwendung von nicht-hydriertem Lecithin mit niedriger Phasenüberganstemperatur als membranbildende Substanz, reicht häufig bereits einfaches Dispergieren aus, ohne dass zusätzlich ein hoher Energieeintrag notwendig wäre. Das Vorliegen einer lamellaren Phase kann, wie vorstehend ausgeführt, von einem Fachmann leicht mittels im Stand der Technik bekannter Verfahren wie z.B. der Polarisationsmikroskopie festgestellt werden.

In einer besonders bevorzugten Ausführungsform umfasst die membranbildende Substanz ein Phospholipid, wie etwa Lecithin oder hydriertes Lecithin, und zusätzlich ein Lipid. Mehr bevorzugt ist das Phospholipid ein Gemisch aus Lecithin und hydriertem Lecithin. In einer besonders bevorzugten Ausführungsform beträgt dabei das Gewichtsverhältnis von Lecithin zu hydriertem Lecithin etwa 10 : 1 bis etwa 1 : 10, mehr bevorzugt etwa 5 : 1 bis etwa 1 : 5 und noch mehr bevorzugt beträgt das Verhältnis von Lecithin zu hydriertem Lecithin etwa 1 : 1. Das zusätzlich zum Phospholipid vorhandene Lipid umfasst in einer bevorzugten Ausführungsform einen flüssigen Wachsester, wie etwa Isopropylmyristat, -palmitat, -stearat oder dergleichen. Ferner können optional weitere Lipide, wie etwa Erdnussöl oder mittelkettige Triglyceride (bevorzugt C₈-C₁₂ Triglyceride), zusätzlich zum Wachsester vorhanden sein. Das Gewichtsverhältnis von Gesamtphospholipid (z.B. etwa Lecithin + hydriertes Lecithin) zu Gesamtlipid (z.B. Wachsester + optionale Triglyceride) beträgt in dieser Ausführungsform bevorzugt etwa 1 : 5 bis etwa 1 : 1, vorzugsweise etwa 1 : 2.

Das Gemisch aus Phospholipid und Lipid wird beispielsweise als Schmelze mit Wasser unter einem hohen Energieeintrag dispergiert. Der hohe Energieeintrag kann durch Ultraschall oder durch Hochdruckhomogenisation erfolgen, wobei Drücke von 50000 bis 250000 Kilopascal (500 bis 2500 bar), vorzugsweise 100000 bis 150000 Kilopascal (1000 bis 1500 bar) zum Einsatz kommen. In der Wasserphase können optional weitere Zusatzstoffe, wie in der vorliegenden Beschreibung beschrieben, wie etwa Glycerol oder Verdickungsmittel (z.B. Xanthangummi und/oder Hydroxypropylmethylcellulose (Hypromellose)), vorhanden sein.

Weitere optionale Inhaltsstoffe sind nachstehend im Zusammenhang mit DMS® Zusammensetzungen genannt. Insbesondere kann die erhaltene Formulierung im Wesentlichen emulgatorfrei, bevorzugt emulgatorfrei sein, d.h. dass in der Formulierung im Wesentlichen keine bzw. keine herkömmlichen Emulgatoren vorliegen, wobei die membranbildende Substanz oder das membranbildende Substanzgemisch nicht als herkömmlicher Emulgator gelten. Beim Dispergieren dieses Gemisches auf die beschriebene Weise wird eine Dispersion erhalten, die zur Ausgestaltung als Schaumformulierung geeignet ist (z.B. unter Verwendung von Treibgasen oder eines Pumpsprays) und die ferner eine lamellar angeordnete Membran ausbildet.

Weitere Basiscremes, die auf die oben beschriebenen "hautähnlichen" Bestandteile zurückgreifen, sind in der Technik auch als DMS® Basiscremes bekannt.

Die DMS® Basiszusammensetzungen können die folgenden Bestandteile Caprylsäure-/Caprinsäuretriglycerid, Sheabutter, Squalan, Ceramid 3, hydriertes Lecithin, Palm Glyceride, Persea Gratissima, Palmöl (Elaesis Guineensis) aufweisen.

Als Stabilisatoren können z.B. Alkohole oder Glykole wie z.B. Pentylenglykol, Caprylylglykol oder Mischungen davon in den DMS® Zusammensetzungen eingesetzt werden.

Eine kommerziell erhältliche DMS® Basis umfasst Caprylsäure-/Caprinsäuretriglycerid, Sheabutter, Squalan, Ceramid 3, hydriertes Lecithin sowie Pentylenglykol.

Eine weitere kommerziell erhältliche DMS® Basis umfasst Caprylsäure-/Caprinsäuretriglycerid, Sheabutter, Squalan, Ceramid 3, hydriertes Lecithin sowie Alkohol.

Eine weitere kommerziell erhältliche DMS® Basis umfasst Caprylsäure-/Caprin-säuretriglycerid, Sheabutter, Squalan, Ceramid 3, hydriertes Lecithin, Persea Gratissima sowie Caprylylglykol.

Eine weitere kommerziell erhältliche DMS® Basis umfasst Caprylsäure-/Caprinsäuretriglycerid, Sheabutter, Squalan, Ceramid 3, hydriertes Lecithin, Palmglyceride, Elaesis Guineensis sowie Pentylenglykol.

Eine bevorzugte DMS® Basis umfasst Caprylsäure-/Caprinsäuretriglycerid, Butyrospermum Parkii, Squalan, Ceramid 3, hydriertes Lecithin sowie Pentylenglykol.

Ein besonders bevorzugtes Caprylsäure-/Caprinsäuretriglycerid ist erhältlich unter der Bezeichnung Miglyol 812 der Firma Sasol und dessen Abmischung mit weiteren Öl- und Wachskomponenten.

Weiterhin sind besonders bevorzugt das Caprylsäure-/Caprinsäuretriglycerid, erhältlich unter der Bezeichnung Miglyol 812 der Firma Sasol /Myritol 312 der Fa. Cognis.

Die erfindungsgemäßen Emulsionen enthalten bevorzugt von 5 bis 50 Gew.-% Ölphase, besonders bevorzugt 10 bis 35 Gew.-% und mehr bevorzugt 15 bis 35 Gew.-% Ölphase. Die Angaben werden jeweils auf das Gesamtgewicht der Emulsion ohne Treibgas bezogen.

Diese Cremezusammensetzungen werden insbesondere bei gereizter, trockener bis sehr trockener, sensitiver bis sehr sensitiver, allergischer und ekzemischer Haut eingesetzt.

Außerdem kann die Ölphase bevorzugt weitere Bestandteile wie z.B. Fettsäuren, insbesondere Stearinsäure oder Öle wie z.B. Cetiol V enthalten.

Bevorzugt wird bei den DMS-Konzentraten und bei den erfindungsgemäßen Formulierungen auf weitere (nicht körperidentische) übliche Hilfsstoffe wie Duftstoffe, Farbstoffe, komedogene Lipide (z.B. Mineralöle) und physiologische Emulgatoren verzichtet, da diese potenziell sensibilisierend sind und zu Irritationen der Haut führen können.

### Wasserphase

Die Wasserphase kann kosmetische Hilfsstoffe enthalten, z.B. niedere Alkohole (z.B. Ethanol, Isopropanol), niedere Diole oder Polyole sowie deren Ether (z.B. Propylenglykol, Glycerin, Butylenglykol, Hexylenglykol und Ethylenglykol), Schaumstabilisatoren und Verdickungsmittel.

Geeignete Verdickungsmittel sind polymere Verdickungsmittel, die teilweise wasserlöslich oder zumindest in Wasser dispergierbar sind und in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie die Beweglichkeit des Wassers einschränken. Geeignete Polymere sind z.B.:
■ Abgewandelte Naturstoffe, wie Celluloseether (z.B. Hydroxypropylcelluloseether, Hydroxyethlylcellulose und Hydroxypropylmethylcelluloseether);
■ Natürliche Verbindungen, wie z.B. Agar-Agar, Carrageen, Polyosen, Stärke, Dextrine, Gelatine, Casein;
■ Synthetische Verbindungen wie z.B. Vinylpolymere, Polyether, Polyimine, Polyamide und Derivate der Polyacrylsäure; und
■ Anorganische Verbindungen wie z.B. Polykieselsäure und Tonmineralien.

Bevorzugt ist ein Celluloseether als Verdickungsmittel in der Formulierung der Erfindung enthalten. Besonderes bevorzugt ist Hydroxypropylmethylcellulose. Eine erfindungsgemäße, bevorzugte Hydroxypropylmethylcellulose ist Metolose 90SH100. Die allgemeine Arzneibuchbezeichnung für Hydroxypropylmethylcellulose ist Hypromellose.

Ein weiteres bevorzugtes Verdickungsmittel ist Xanthan Gummi, insbesondere Keltrol® CG Xanthan Gummi.

Die Hydroxypropylmethylcellulose und Xanthan Gummi können ebenfalls nebeneinander in der erfindungsgemäßen Formulierung eingesetzt werden.

Die erfindungsgemäßen Emulsionen enthalten bevorzugt von 0,2 bis 3,0 Gew.-% Verdickungsmittel (bezogen auf das Trockengewicht des Verdickungsmittels und das Gesamtgewicht der Emulsion ohne Treibgas). Besonders bevorzugt sind 0,5 bis 2,5 Gew.-% Verdickungsmittel.

### Wirkstoffe

Der enthaltene Wirkstoff kann unter allen oberflächig auf der Haut applizierbaren Wirkstoffen und Mischungen dieser gewählt werden. Der Wirkstoff kann kosmetisch oder pharmazeutisch wirken. Entsprechend erhält man kosmetische oder dermatologische (als Medizinprodukt oder Arzneimittelprodukt einzusetzende) Schaumformulierungen. Ferner kann die Formulierung zum Schutz der Haut vor Umwelteinflüssen dienen. Der Wirkstoff kann rein pflanzlich oder synthetisch sein. Die Gruppe der Wirkstoffe kann sich auch mit den anderen Inhaltstoffgruppen, wie z.B. der Ölkomponente, den Verdickungsmitteln oder den Feststoffemulgatoren, überschneiden. Beispielsweise können manche Ölkomponenten auch als Wirkstoffe dienen, wie z.B. Öle mit mehrfach ungesättigten Fettsäuren, oder Feststoffemulgatoren, wie z.B. partikuläres Titandioxid als UV-Filter dienen kann. Je nach Eigenschaftsprofil sind die Substanzen mehreren Gruppen zuzuordnen.

Wirkstoffe der erfindungsgemäßen Formulierungen werden vorteilhaft gewählt aus der Gruppe der Substanzen mit feuchtigkeitspendenden und barrierestärkenden Eigenschaften, wie z. B. Hydroviton, eine Nachbildung des NMF, Pyrrolidoncarbonsäure und deren Salze, Milchsäure und deren Salze, Glycerol, Sorbitol, Propylenglykol und Harnstoff, Substanzen aus der Gruppe der Proteine und Proteinhydrolysate wie z. B. Collagen, Elastin sowie Seidenprotein, Substanzen aus der Gruppe der Glucosaminoglucane, wie z. B. Hyaluronsäure, aus der Gruppe der Kohlenhydrate, wie z. B. Pentavitin, das in seiner Zusammensetzung dem Kohlehydratgemisch der menschlichen Hornschicht entspricht, und der Gruppe der Lipide und Lipidvorstufen wie beispielsweise die Ceramide. Weitere vorteilhafte Wirkstoffe können im Sinne der vorliegenden Erfindung ferner gewählt werden aus der Gruppe der Vitamine, wie z. B. Panthenol, Niacin, α-Tocopherol und seine Ester, Vitamin A sowie Vitamin C. Außerdem können die Wirkstoffe, gewählt aus der Gruppe der Antioxidantien z. B. Galate und Polyphenole, verwendet werden. Harnstoff, Hyaluronsäure und Pentavitin sind bevorzugte Substanzen.

Es ist ferner bevorzugt, dass als Wirkstoffe Substanzen mit hautberuhigender und regenerierender Wirkung eingesetzt werden, wie z. B. Panthenol, Bisabolol und Phytosterole.

Vorteilhafte Wirkstoffe im Sinne der vorliegenden Erfindung sind auch Pflanzen und Pflanzenextrakte. Hierzu gehören z.B. Algen, Aloe, Arnika, Bartflechten, Beinwell, Birke, Brennessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss sowie deren Extrakte.

Die erfindungsgemäßen Zubereitungen können ferner als Wirkstoffe Antimykotika und Antiseptika/Desinfzientia synthetischen oder natürlichen Ursprungs enthalten.

Weitere Wirkstoffe sind Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, Wundheilungsfördernde Wirkstoffe, z.B. Wachstumsfaktoren, Enzympräparate und Insektizide.

### Weitere Bestandteile der Emulsion

Die Formulierungen können außerdem optional Farbstoffe, Perlglanzpigmente, Duftstoffe/Parfum, Lichtschutzfiltersubstanzen, Konservierungsmittel, Komplexbildner, Antioxidantien und Repellentien sowie pH-Wert Regulatoren enthalten. In einer bevorzugten Ausführungsform sind die Formulierungen der Erfindung jedoch frei von Bestandteilen, die zu Irritationen der Haut führen können, insbesondere frei von Duftstoffen/Parfum, Farbstoffen und herkömmlichen Emulgatoren.

Die erfindungsgemäßen Schaumformulierungen können neben den bereits oben genannten Bestandteilen weitere natürliche Fette, wie z.B. Sheabutter, Neutralöle, Olivenöl, Squalan, Ceramide und Feuchthaltesubstanzen enthalten, wie sie in der Technik üblich sind.

Die obige Aufzählung der einzelnen Bestandteile der Emulsion soll so verstanden werden, dass einzelne Beispielkomponenten aufgrund ihrer verschiedenen Eigenschaften auch mehreren Gruppen zugeordnet werden können.

### Treibgase

Geeignete Treibgase sind z.B. N₂O, Propan, Butan und i-Butan, Die fertige Schaumformulierung enthält von etwa 5 bis etwa 15 Gew.-%, bevorzugt etwa 10 Gew.-% Treibgas.

### Herstellungsverfahren

Die erfindungsgemäßen Schaumformulierungen werden durch Bereitstellen einer Emulsion, bevorzugt vom Typ Öl-in-Wasser und Abfüllen der Emulsion und gegebenenfalls Beaufschlagen mit Treibgas in einen geeigneten Behälter wie z.B. einen Druckbehälter bereitgestellt. Alternativ zum Treibgas und Druckbehälter kann die Emulsion auch in einen anderen Behälter abgefüllt werden, der auch ohne Treibgas dafür geeignet ist, die Emulsion als Schaum abzugeben. Solche Systeme sind dem Fachmann bekannt.

Insbesondere werden die erfindungsgemäßen Emulsionen durch ein Verfahren hergestellt, das die folgenden Schritte umfasst:
(1) Bereitstellen einer Ölphase gegebenenfalls umfassend mindestens eine membranbildende Substanz, die in der Formulierung eine lamellare Membran ausbildet,
(2) Bereitstellen einer Wasserphase,
(3) Zusammengeben und Homogenisieren wie z.B. durch Ultraschall oder Hochdruckhomogenisation der beiden Phasen,
(4) Optional Hinzufügen mindestens einer oder mindestens einer weiteren membranbildenden Substanz,
(5) Optional Homogenisieren wie z.B. durch Ultraschall oder Hochdruckhomogenisation , um eine Emulsion zu erhalten, wobei in mindestens einem der Schritte (1) oder (4) mindestens eine membranbildende Substanz enthalten ist, die in der Formulierung eine lamellare Membran ausbildet.

Bevorzugt werden die Ölphase und die Wasserphase jeweils bei einer Temperatur im Bereich von etwa 40 bis etwa 90 °C vermischt und homogenisiert, besonders bevorzugt ist ein Temperaturbereich von etwa 60 bis etwa 80 °C, mehr bevorzugt eine Temperatur von etwa 70 °C.

Für das Homogenisieren kann jedes Mittel oder Verfahren, welches in der Technik bekannt ist, verwendet werden. Bevorzugt werden die Phasen mit einem hochtourigen Rührwerk homogenisiert. In einer bevorzugten Ausführungsform erfolgt das Homogenisieren mittels Hochdruckhomogenisation. In einer anderen Ausführungsform wird das Homogenisieren mit Hilfe von Ultraschall unterstützt.

In einem bevorzugten Herstellungsverfahren wird die Ölphase in die Wasserphase eingerührt und homogenisiert. Falls erforderlich wird die Emulsion unter Rühren auf Raumtemperatur abgekühlt. In einem besonders bevorzugten Verfahren wird zu dieser Mischung eine geeignete Menge eines DMS® Konzentrats hinzugegeben und das Konzentrat in die vorliegende Emulsion eingearbeitet. Das Verfahren, das im folgenden beschrieben wird, kann ebenso mit anderen lamellaren Phasen als dem DMS® Konzentrat durchgeführt werden.

Das DMS® Konzentrat kann bereits vor dem Homogenisieren mit der Wasserphase in die Ölphase gegeben werden oder nach dem Homogenisieren der Öl- und Wasserphase zu der Mischung hinzugegeben werden. Es ist bevorzugt, dass das DMS® Konzentrat nach dem ersten Homogenisierungsschritt zu der Mischung hinzugegeben und die Mischung homogenisiert wird.

Wenn die Emulsion ein Verdickungsmittel umfasst, umfasst das Verfahren vorteilhaft die folgenden weiteren Schritte:
(6) Bereitstellen einer wässrigen Verdickungsmittellösung
(7) Vermischen der Verdickungsmittellösung mit der Emulsion

Bevorzugt wird die erfindungsgemäße Emulsion mit etwa 10 Gew.-% Treibgas beaufschlagt.

### Verwendungen

Die Schaumformulierungen der vorliegenden Erfindung können für alle kosmetischen und dermatologischen (als Medizinprodukt oder Arzneimittel) Zwecke eingesetzt werden. Zum Beispiel könne die Formulierungen als Hautpflegemittel oder Hautreinigungsmittel eingesetzt werden. Sie können ferner als Träger für Wirkstoffe dienen und im medizinisch dermatologischen Bereich eingesetzt werden. Insbesondere können die Formulierungen als Sonnenschutzmittel eingesetzt werden.

### Beispiele

### Zusammensetzung der Schaumformulierung

### a) Wasserphase

Die Wasserphase wird durch Mischen der Bestandteile hergestellt.

| Bestandteil | Menge |
|---|---|
| HPMC (Metolose 90SH100) | 1,5 g |
| Xanthan Gummi (Keltrol® CG) | 0,5 g |
| Wasser | 78 g |

### b) Ölphase

### Beispiel 1

| Bestandteil | Beispiel 1 |
|---|---|
| DMS Konzentrat | 5 g |
| Miglyol 812 | 14 g |
| Stearinsäure | 1 g |
| Wasserphase | ad 100 g |

### Beispiel 2

| Bestandteil | Beispiel 2 |
|---|---|
| DMS Konzentrat | 5 g |
| Miglyol 812 | 14 g |
| Stearinsäure | 1 g |
| Cetiol V | 5 g |
| Wasserphase | ad 100 g |

Die Stearinsäure wird unter Erwärmen auf ca. 70 °C in Miglyol 812 (Beispiel 1) bzw. in der Mischung aus Miglyol 812 und Cetiol V (Beispiel 2) gelöst.

Diese Ölphase wird in die Wasserphase eingerührt und mit einem hochtourigen Rührwerk homogenisiert. Die resultierende Emulsion wird unter Rühren auf Raumtemperatur abgekühlt und das DMS®-Konzentrat mit einem hochtourigen Rührwerk eingearbeitet.

Das verwendete DMS® Konzentrat weist die folgende INCI-Bestandteile auf:
Aqua (and) Hydrogenated Lecithin (and) Caprylic/Capric Triglyceride(and) Pentylene Glycol (and) Butyrospermum Parkii (and) Glycerin (and) Squalane (and) Ceramide 3

### Herstellung der Schaumformulierung

90 g der so hergestellte Emulsion werden in Aerosolbehälter abgefüllt und nach deren Verschließen mit einem Ventildeckel mit 10 g Treibmittel beaufschlagt.

### Beispiel 3

| Bestandteil | Beispiel 3 |
|---|---|
| Sojalecithin | 3,0 g |
| Sojalecithin, hydriert | 3,0 g |
| Mittelkettige Triglyceride | 7,0 g |
| Isopropylmyristat | 7,0 g |
| Xanthan Gummi | 0,4 g |
| Hypromellose | 1,2 g |
| Glycerol 85% | 5,0 g |
| Wasser | 73,4 g |

### Herstellung

Das Gemisch aus Sojalecithin und hydriertem Sojalecithin (z.B. Phospholipon 80 H und Phospholipon 85 G) werden in einer Mischung aus mittelkettigen Triglyceriden (z.B. Miglyol 812) und Isopropylmyristat bei 60 °C gelöst. Bei hohem Energieeintrag (z.B. Ultraschall oder Hochdruckhomogenisation) wird die Lipidschmelze in einer Mischung aus Wasser und Glycerol dispergiert (Hochdruckhomogenisator: Avestin Emulsiflex-C3; Druck: 1400 bar). Anschließend wird die Lösung von Xanthan Gummi (z.B. Keltrol CG) und Hypromellose (z.B. Metolose 90SH100) in Wasser unter Rühren zugemsicht.

### Herstellung der Schaumformulierung

90 g der so hergestellte membranbildenden Emulsion werden in Aerosolbehälter abgefüllt und nach deren Verschließen mit einem Ventildeckel mit 10 g Treibmittel beaufschlagt.

### Schaumerzeugung

Ein stabiler, feinblasiger Cremeschaum entsteht bei der Entnahme der Schaumformulierung aus der Druckgaspackung mittels eines geeigneten Ventils mit aufgesetztem Schaumapplikator. Die Struktur des Cremeschaumes bleibt für einen Zeitraum erhalten, der ausreicht, den Schaum auf der Haut gleichmäßig zu verteilen.

### Weitere Gegenstände der Erfindung

1. Schaumformulierung umfassend eine Emulsion, umfassend eine Ölphase und eine Wasserphase, wobei die Ölphase mindestens eine membranbildende Substanz umfasst, die in der Schaumformulierung eine lamellar angeordnete Membran ausbildet.
2. Schaumformulierung gemäß Gegenstand 1, wobei die Emulsion eine Öl-in-Wasser Emulsion ist.
3. Schaumformulierung gemäß einem der Gegenstände 1-2, wobei die mindestens eine membranbildende Substanz ein Lipid, bevorzugt ein Triglycerid umfasst.
4. Schaumformulierung gemäß Gegenstand 3, wobei das Triglycerid Caprylsäure/Caprinsäuretriglycerid umfasst.
5. Schaumformulierung gemäß einem der Gegenstände 1-4, wobei das Lipid ferner ein Phospholipid, vorzugsweise Lecithin umfasst.
6. Schaumformulierung gemäß Gegenstand 5, wobei das Lecithin ein hydriertes Lecithin umfasst.
7. Schaumformulierung gemäß einem der Gegenstände 1-6, wobei die Emulsion ferner mindestens ein Verdickungsmittel umfasst, welches bevorzugt ausgewählt wird aus der Gruppe bestehend aus Hydroxypropylmethylcellulose, Xanthan Gummi und Mischungen der vorgenannten.
8. Schaumformulierung gemäß einem der Gegenstände 1-7, wobei die Emulsion ferner einen Stabilisator umfasst.
9. Schaumformulierung gemäß Gegenstand 8, wobei der Stabilisator Pentylenglykol umfasst.
10. Schaumformulierung gemäß einem der Gegenstände 1-9, wobei die Emulsion ferner weitere übliche Bestandteile wie Sheabutter, Glycerin, Squalan, Ceramid, bevorzugt Ceramid 3 oder Mischungen der vorgenannten umfasst.
11. Schaumformulierung gemäß einem der Gegenstände 1-10, wobei die Emulsion ferner weitere übliche Bestandteile wie Öle und fettende Substanzen umfasst.
12. Schaumformulierung gemäß einem der Gegenstände 1-11, wobei die Emulsion ferner mindestens einen Wirkstoff umfasst.
13. Schaumformulierung gemäß Gegenstand 12, wobei der Wirkstoff ausgewählt wird aus der Gruppe bestehend aus Hydroviton, Pyrrolidoncarbonsäure und deren Salze, Milchsäure und deren Salze, Glycerol, Sorbitol, Propylenglykol, Harnstoff, Collagen, Elastin, Seidenprotein, Hyaluronsäure, Pentavitin, Ceramide, Panthenol, Niacin, α-Tocopherol und seine Ester, Vitamin A, Vitamin C, Galate, Polyphenole, Panthenol, Bisabolol, Phytosterole, Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, Wundheilungsfördernde Wirkstoffe, Wachstumsfaktoren, Enzympräparate, Insektizide und Pflanzenmaterial wie, bzw. Pflanzenextrakte von, Algen, Aloe, Arnika, Bartflechten, Beinwell, Birke, Brennnessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss oder Mischungen davon, ausgewählt ist.
14. Schaumformulierung gemäß einem der Gegenstände 1-13, wobei die Emulsion eine im Wesentlichen emulgatorfreie Emulsion ist.
15. Schaumformulierung gemäß Gegenständen 1-14, wobei die Emulsion emulgatorfrei ist.
16. Schaumformulierung gemäß Gegenständen 3-15, wobei das Lipid körperidentische Fette umfasst.
17. Schaumformulierung gemäß einem der Gegenstände 1-16, wobei die Formulierung eine Schaumcreme ist.
18. Schaumformulierung gemäß einem der Gegenstände 1-17, wobei die Emulsion mindestens ein Phospholipid und mindestens einen flüssigen Wachsester, vorzugsweise im Gewichtsverhältnis Phospholipid:Wachsester von 1:5 bis 1:1, mehr bevorzugt 1:2, umfasst.
19. Schaumformulierung gemäß Gegenstand 18, wobei das Phospholipid Lecithin umfasst, vorzugsweise ein Gemisch aus Lecithin und hydriertem Lecithin umfasst, bevorzugt im Gewichtsverhältnis von etwa 10:1 bis 1:10, mehr bevorzugt etwa 5:1 bis 1:5, noch mehr bevorzugt etwa 1:1; und/oder wobei der Wachsester Isopropylmyristat umfasst ist; und/oder wobei weiterhin ein Triglycerid, vorzugsweise ein C₈-C₁₂ Triglycerid, enthalten ist.
20. Schaumformulierung gemäß einem der Gegenstände 1-19, wobei die membranbildende Substanz in Wasser unlöslich ist.
21. Schaumformulierung gemäß einem der Gegenstände 1-20, wobei die membranbildende Substanz einen HLB-Wert von größer als 8, vorzugsweise zwischen 9 bis 11, mehr bevorzugt zwischen 9,5 bis 10,5 aufweist.
22. Verwendung einer Schaumformulierung gemäß einem der Gegenstände 1-21 als Hautpflegemittel.
23. Verwendung einer Schaumformulierung gemäß einem der Gegenstände 1-21 als Hautreinigungsmittel.
24. Verwendung einer Schaumformulierung gemäß einem der Gegenstände 1-21 als Sonnenschutzmittel.
25. Verwendung einer Schaumformulierung gemäß einem der Gegenstände 1-21 zur Herstellung eines Kosmetikums, Medizinproduktes oder Arzneimittels.
26. Verfahren zur Herstellung einer Schaumformulierung umfassend die folgenden Schritte:
   a) Herstellen einer Emulsion bevorzugt vom Typ Öl in Wasser
   b) Abfüllen der Emulsion und Treibgas in einen Druckbehälter, oder
   c) Abfüllen der Emulsion in einen anderen als einen Druckbehälter, der bei Abgabe der Emulsion einen Schaum erzeugt.
27. Verfahren gemäß Gegenstand 26 zur Herstellung einer Schaumformulierung nach einem der Gegenstände 1 bis 21.
28. Verfahren gemäß Gegenstand 27, wobei das Herstellen der Emulsion die Schritte umfasst:
   (1) Bereitstellen einer Ölphase gegebenenfalls umfassend mindestens eine membranbildende Substanz, die in der Formulierung eine lamellare Membranen ausbildet,
   (2) Bereitstellen einer Wasserphase,
   (3) Zusammengeben und Homogenisieren der beiden Phasen,
   (4) Optional Hinzufügen mindestens einer oder mindestens einer weiteren membranbildenden Substanz,
   (5) Optional Homogenisieren, um eine Emulsion zu erhalten,
   wobei in mindestens einem der Schritte (1) oder (4) mindestens eine membranbildende Substanz umfasst ist, die in der Formulierung eine lamellare Membran ausbildet.
29. Verfahren gemäß Gegenstand 28, wobei die Ölphase und die Wasserphase bei einer Temperatur zwischen etwa 40 bis etwa 90 °C, bevorzugt zwischen etwa 60 bis etwa 80 °C, mehr bevorzugt etwa 70 °C homogenisiert werden.
30. Verfahren gemäß Gegenstand 28 oder 29, wobei die Emulsion ein Verdickungsmittel umfasst, ferner umfassend die Schritte:
   (6) Bereitstellen einer wässrigen Verdickungsmittellösung
   (7) Mischen der Verdickungsmittellösung mit der Emulsion.
31. Verfahren gemäß einem der Gegenstände 28 bis 30, wobei die Schaumformulierung 10 Gew-% Treibgas enthält.
32. Verfahren gemäß einem der Gegenstände 28 bis 30, wobei das Homogenisieren unter hohem Energieeintrag erfolgt, vorzugsweise mittels Hochdruckhomogenisation und/oder Ultraschall.
33. Verfahren gemäß einem der Gegenstände 28-30 und 32, wobei das Homogenisieren bei einem Druck von etwa 50.000 bis etwa 250.000 Kilopascal, vorzugsweise bei etwa 100.000 bis 150.000 Kilopascal durchgeführt wird.

## Patentansprüche

1. Schaumformulierung umfassend eine Emulsion, umfassend eine Ölphase und eine Wasserphase, wobei die Ölphase mindestens eine membranbildende Substanz umfasst, die in der Schaumformulierung eine lamellar angeordnete Membran ausbildet.

2. Schaumformulierung gemäß Anspruch 1, wobei die Emulsion eine Öl-in-Wasser Emulsion ist.

3. Schaumformulierung gemäß einem der Ansprüche 1-2, wobei die mindestens eine membranbildende Substanz ein Lipid, bevorzugt ein Triglycerid umfasst, wobei das Triglycerid vorzugsweise Caprylsäure/Caprinsäuretriglycerid umfasst.

4. Schaumformulierung gemäß einem der Ansprüche 1-3, wobei das Lipid ferner ein Phospholipid, vorzugsweise Lecithin und besonders bevorzugt hydriertes Lecithin umfasst.

5. Schaumformulierung gemäß einem der Ansprüche 1-4, wobei die Emulsion ferner mindestens ein Verdickungsmittel umfasst, welches bevorzugt ausgewählt wird aus der Gruppe bestehend aus Hydroxypropylmethylcellulose, Xanthan Gummi und Mischungen der vorgenannten.

6. Schaumformulierung gemäß einem der Ansprüche 1-5, wobei die Emulsion ferner einen Stabilisator, vorzugsweise Pentylenglykol umfasst.

7. Schaumformulierung gemäß einem der Ansprüche 1-6, wobei die Emulsion ferner weitere übliche Bestandteile wie Sheabutter, Glycerin, Squalan, Ceramid, bevorzugt Ceramid 3, Öle und fettenden Substanzen oder Mischungen der vorgenannten umfasst.

8. Schaumformulierung gemäß einem der Ansprüche 1-7, wobei die Emulsion ferner mindestens einen Wirkstoff umfasst, vorzugsweise ausgewählt aus der Gruppe bestehend aus Hydroviton, Pyrrolidoncarbonsäure und deren Salze, Milchsäure und deren Salze, Glycerol, Sorbitol, Propylenglykol, Harnstoff, Collagen, Elastin, Seidenprotein, Hyaluronsäure, Pentavitin, Ceramide, Panthenol, Niacin, α-Tocopherol und seine Ester, Vitamin A, Vitamin C, Galate, Polyphenole, Panthenol, Bisabolol, Phytosterole, Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, Wundheilungsfördernde Wirkstoffe, Wachstumsfaktoren, Enzympräparate, Insektizide und Pflanzenmaterial wie, bzw. Pflanzenextrakte von, Algen, Aloe, Arnika, Bartflechten, Beinwell, Birke, Brennnessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss oder Mischungen davon.

9. Schaumformulierung gemäß einem der Ansprüche 1-8, wobei die Emulsion eine im Wesentlichen, vorzugsweise völlig, emulgatorfreie Emulsion ist.

10. Schaumformulierung gemäß Anspruch 3-9, wobei das Lipid körperidentische Fette umfasst.

11. Schaumformulierung gemäß einem der Ansprüche 1-10, wobei die Formulierung eine Schaumcreme ist.

12. Schaumformulierung gemäß einem der Ansprüche 1-11, wobei die Emulsion mindestens ein Phospholipid und mindestens einen flüssigen Wachsester, vorzugsweise im Gewichtsverhältnis Phospholipid:Wachsester von 1:5 bis 1:1, mehr bevorzugt 1:2, umfasst, wobei das Phospholipid vorzugsweise Lecithin und besonders bevorzugt ein Gemisch aus Lecithin und hydriertem Lecithin umfasst, bevorzugt im Gewichtsverhältnis von etwa 10:1 bis 1:10, mehr bevorzugt etwa 5:1 bis 1:5, noch mehr bevorzugt etwa 1:1; und/oder wobei der Wachsester Isopropylmyristat umfasst; und/oder wobei weiterhin ein Triglycerid, vorzugsweise ein C₈-C₁₂ Triglycerid, enthalten ist.

13. Schaumformulierung gemäß einem der Ansprüche 1-12, wobei die membranbildende Substanz in Wasser unlöslich ist, und bevorzugt einen HLB-Wert von größer als 8, vorzugsweise zwischen 9 bis 11, mehr bevorzugt zwischen 9,5 bis 10,5 aufweist.

14. Verwendung einer Schaumformulierung gemäß einem der Ansprüche 1-13 als Hautpflegemittel, Hautreinigungsmittel oder als Sonnenschutzmittel.

15. Verwendung einer Schaumformulierung gemäß einem der Ansprüche 1-13 zur Herstellung eines Kosmetikums, Medizinproduktes oder Arzneimittels.

16. Verfahren zur Herstellung einer Schaumformulierung umfassend die folgenden Schritte:
a) Herstellen einer Emulsion bevorzugt vom Typ Öl in Wasser
b) Abfüllen der Emulsion und Treibgas in einen Druckbehälter, oder
c) Abfüllen der Emulsion in einen anderen als einen Druckbehälter, der bei Abgabe der Emulsion einen Schaum erzeugt,
wobei das Herstellen der Emulsion vorzugsweise die Schritte umfasst:
(1) Bereitstellen einer Ölphase gegebenenfalls umfassend mindestens eine membranbildende Substanz, die in der Formulierung eine lamellare Membranen ausbildet,
(2) Bereitstellen einer Wasserphase,
(3) Zusammengeben und Homogenisieren der beiden Phasen,
(4) Optional Hinzufügen mindestens einer oder mindestens einer weiteren membranbildenden Substanz,
(5) Optional Homogenisieren, um eine Emulsion zu erhalten,
wobei in mindestens einem der Schritte (1) oder (4) mindestens eine membranbildende Substanz umfasst ist, die in der Formulierung eine lamellare Membran ausbildet, und wobei vorzugsweise die Ölphase und die Wasserphase bei einer Temperatur zwischen etwa 40 bis etwa 90 °C, bevorzugt zwischen etwa 60 bis etwa 80 °C, mehr bevorzugt etwa 70 °C homogenisiert werden.

17. Verfahren gemäß Anspruch 16, wobei das Homogenisieren unter hohem Energieeintrag erfolgt, vorzugsweise mittels Hochdruckhomogenisation und/oder Ultraschall, vorzugsweise mittels Homogenisieren bei einem Druck von etwa 50.000 bis etwa 250.000 Kilopascal, vorzugsweise bei etwa 100.000 bis 150.000 Kilopascal.
